# EUROPEAN PATENT APPLICATION

(11) **EP 4 741 413 A1**
(43) Date of publication of application: **13.05.2026**
(21) Application number: 24852325.0
(22) Date of filing: 07.08.2024
(51) Int. Cl.: C07K 7/06, A61K 9/00, A61P 27/02, A61P 27/04, A61K 38/00

(54) **PEPTIDE HAVING ACTIVITY OF ALLEVIATING DRY EYE DISEASE AND USES THEREOF**

(30) Priority: 10.08.2023 KR 20230104783
(71) Applicant: Caregen Co., Ltd., Seoul 06188 (KR)
(72) Inventor: CHUNG, Yong Ji, Seoul 06188 (KR); KIM, Eun Mi, Seoul 06188 (KR); LEE, Eung Ji, Seoul 06188 (KR)
(74) Representative: Bandpay & Greuter
(86) International application number: PCT/KR2024/011676
(87) International publication number: WO 2025/033971

(57) **Abstract**

The peptide of the present disclosure has a binding capacity to PAC1R, promotes activation of a PAC1R signaling pathway, and induces neuroblast differentiation, thereby having a tear secretion promoting activity. In addition, the peptide of the present disclosure promotes expression and secretion of mucin which is a tear component in conjunctival goblet cells, and has an activity of inhibiting inflammatory response and cell damage in human corneal epithelial cells. The peptide of the present disclosure may be used as an active material for treatment, prevention, and improvement of dry eye disease.

## Description

### TECHNICAL FIELD

### [CROSS-REFERENCE TO RELATED APPLICATION]

This application claims the benefit of priority to Korean Patent Application No. 10-2023-0104783, filed in the Korean Intellectual Property Office on August 10, 2023, the entire contents of which are incorporated herein by reference.

### [TECHNICAL FIELD]

The present disclosure relates to a novel peptide having an activity of improving dry eye disease and a use thereof.

### BACKGROUND ART

Dry eye disease (dry eye syndrome) refers to a condition or disease in which dry symptoms occur in the cornea or conjunctiva of the eye due to decreased tear secretion or changes in tear components even in the case of normal tear secretion. Causes of dry eye disease are very diverse, for example, a decreased tear secretion or changes in tear components due to aging, decreased tear production due to diseases such as rheumatoid arthritis, Sjogren syndrome, lupus, scleriasis, diabetes, and vitamin A-deficiency, reduced mucus secretion due to chronic conjunctivitis, excessive tear evaporation due to hyperthyroidism, decreased tear production due to decreased female hormones, evaporation of tears due to taking medicines such as antibiotics and antihistamines, eye irritation from dry environments, smoke, dust, sunlight, wind, and the like, a decreased number of eye blinks, and the like.

The dry eye disease starts from desiccating stress on the cornea and conjunctiva of the eye, and the expression of pro-inflammatory cytokines such as TNF-α, IL-1β, and IL-6 is increased due to the desiccating stress. Accordingly, antigen presenting cells (APCs) around the cornea and conjunctival tissue of the eye are activated and then move to lymph nodes and induce differentiation of Th1 and Th17 cells. The differentiated Th1 and Th7 cells move to a lesion area again and promote the secretion of pro-inflammatory cytokines such as TNF-α, IL-1β, IL-17, and IFN-γ to further intensify inflammation, and damages such as matrix degradation is induced by matrix metalloproteinases (MMPs) increased by the stimulation (Mucosal Immunol. 2009 Jul; 2(4): 375-6. Role of Th17 cells in the immunopathogenesis of dry eye disease).

Pituitary adenylate cyclase-activating polypeptide (PACAP) is a peptide hormone in the body and as its receptor, VPAC1, VPAC2, and PAC1 receptors are known, and recently, the results of confirming an effect of improving dry eye disease by promoting tear secretion in lacrimal gland by PACAP/PAC1R mechanism activation in the dry eye disease animal model have been reported (Int J Mol Sci. 2022 Jan 8; 23(2): 664., Potential Therapeutic Role of Pituitary Adenylate Cyclase-Activating Polypeptide for Dry Eye Disease). In addition, the results of confirming that synthetic peptide derived from PACAP having a PAC1R-specific agonist activity has an activity of healing corneal damage and increasing tear secretion from the lacrimal gland have been reported (Invest Ophthalmol Vis Sci. 2015 Jul; 56(8): 4336-49., A New Recombinant PACAP-Derived Peptide Efficiently Promotes Corneal Wound Repairing and Lacrimal Secretion). Until now, PACAP has been confirmed to involve in improving symptoms of dry eye disease through an anti-inflammatory activity, a corneal damage inhibition activity, and a tear secretion inducing activity.

### [Related Art Document]

### [Non-patent Document]

Mucosal Immunol. 2009 Jul;2(4):375-6.
Int J Mol Sci. 2022 Jan 8;23(2) :664.
Invest Ophthalmol Vis Sci. 2015 Jul;56(8):4336-49.

### DISCLOSURE OF THE INVENTION

### TECHNICAL PROBLEM

The present inventors studied and made effort in order to develop a stable and effective active material having an activity of improving dry eye disease. As a result, it was experimentally confirmed that a novel peptide synthesized by the present inventors has an excellent activity of improving dry eye disease through an activity of promoting tear secretion, an activity of promoting secretion of mucin which is a tear film component, an activity of preventing damage to corneal epithelial cells, and the like, thereby completing the present disclosure.

Therefore, an aspect of the present disclosure provides a novel peptide having an activity of improving dry eye disease.

Another aspect of the present disclosure provides a pharmaceutical composition for treating or preventing dry eye disease including the peptide as an effective component.

### TECHNICAL SOLUTION

In order to accomplish the object of the present invention,

The present disclosure provides a peptide comprising an amino acid sequence disclosed in SEQ ID NO: 1.

The present disclosure provides a pharmaceutical composition for treating or preventing dry eye disease comprising the peptide as an effective component.

Hereinafter, the present disclosure will be described in detail.

### Peptide and its activity

According to an aspect of the present disclosure, a peptide comprising an amino acid sequence disclosed in SEQ ID NO: 1 is provided.

[Amino acid sequence of SEQ ID NO: 1]
TLKKNKRFSK

The term "peptide" in the present specification means a linear molecule formed by amino acid residues bonded to each other by a peptide bond.

A peptide including an amino acid sequence of SEQ ID NO: 1 of the present disclosure may be used without modification, but variants or fragments of the amino acid having different sequences may be used by deletion, insertion, or substitution of an amino acid residue or a combination thereof, within a range which does not affect the natural activity of the peptide, for example, the activity of improving dry eye disease.

The peptide of the present disclosure may be modified by phosphorylation, sulfation, acrylation, glycosylation, methylation, farnesylation, and the like, within a range which does not change the activity.

The peptide of the present disclosure includes a peptide including substantially the same amino acid sequence as the peptide including the amino acid sequence of SEQ ID NO: 1 and variants thereof or activity fragments thereof. The substantially the same amino acid sequence refers to an amino acid sequence having sequence identity of 75% or more, for example, 80% or more, 85% or more, 90% or more, 95% or more, 97% or more, or 98% or more with the amino acid sequence of SEQ ID NO: 1. In addition, the peptide may further include an amino acid sequence prepared for a specific purpose for increasing targeting sequence, tag, labeled residue, half-life, or peptide stability.

The peptide of the present disclosure may be obtained by selecting a portion of the amino acid sequence and inducing N-terminal and/or C-terminal modification for increasing the activity. The stability of the peptide of the present disclosure may be significantly improved by the N-terminal and/or C-terminal modification, and for example, when the peptide is administered in vivo, a half life may be increased. The term "stability" includes the meanings of not only stability in vivo for protecting the peptide of the present disclosure from attack by protein-cleaving enzymes in vivo, but also storage stability (for example, storage stability at room temperature).

The N-terminal modification may be the N-terminal of peptide to which a protecting group selected from the group consisting of an acetyl group, a fluoreonylmethoxycarbonyl group, a formyl group, a palmitoyl group, a myristyl group, a stearyl group, and a polyethylene glycol (PEG) is bonded. The C-terminal modification may be the C-terminal of peptide to which a hydroxyl group (-OH), an amino group (-NH₂), azide (-NHNH₂), and the like are bonded, but is not limited thereto.

The peptide of the present disclosure may be prepared by various methods well known in the art to which the present disclosure pertains. For example, the peptide of the present disclosure may be prepared by a chemical synthesis method known in the art, in particular, solid-phase synthesis techniques (Merrifield, J. Amer. Chem. Soc. 85:2149-54(1963); Stewart, et al., Solid Phase Peptide Synthesis, 2nd. ed., Pierce Chem. Co.: Rockford, 111(1984)) or liquid synthesis technique (US Patent Registration No. 5,516,891).

The peptide of the present disclosure has an activity of improving, treating, or preventing dry eye disease.

In an exemplary embodiment, the peptide of the present disclosure has the following activities related to the tear secretion promoting activity:
(i) having a binding capacity to a pituitary adenylate cyclase 1 receptor (PAC1R) and promoting activation of PAC1R signaling pathway,
(ii) promoting movement of Aquaporin 5 (AQP5) protein to a plasma membrane, and
(iii) inducing neuroblast differentiation.

In an exemplary embodiment, the peptide of the present disclosure has an activity of promoting the expression and secretion of mucin which is a component of tears in conjunctival goblet cells.

In an exemplary embodiment, the peptide of the present disclosure has an activity of inhibiting inflammatory response and cell damage which are induced by IFN-γ in human corneal epithelial cells.

In an exemplary embodiment, the peptide of the present disclosure has an activity of inhibiting inflammatory response and cell damage which are induced by ultraviolet (UVB) irradiation in human corneal epithelial cells.

### Composition for treating or preventing dry eye disease

According to another aspect of the present disclosure, a pharmaceutical composition for treating or preventing dry eye disease comprising a peptide comprising an amino acid sequence disclosed in SEQ ID NO: 1 as an effective component is provided.

The term "dry eye disease" used in the present specification refers to eye disease which causes ocular surface damage and feeling irritating symptoms such as visual impairment, sore eyes, irritation, foreign body sensation, or dryness, due to lack of tears, excessive evaporation of tears, instability of a tear film, or imbalance of tear components.

The term "dry eye disease" used in the present specification may include one or more disorder characterized by insufficient or defective tears, for example, the following disorders, but is not necessarily limited to the following disorders or diseases: dry eye, dry eye syndrome, dry eye disease, evaporative dry eye, water-deficient dry eye, dry keratitis (corneal dryness and inflammatory disease), keratoconjunctivitis sicca (dryness affecting both cornea and conjunctiva), and dysfunctional tear syndrome (inadequate tear quality or quantity).

The term "treatment" used in the present specification refers to prevention, elimination, cure, suppression, and reduction of at least one symptom of condition, disease, or disorder.

The pharmaceutical composition of the present disclosure may include a therapeutically effective amount of a peptide comprising an amino acid sequence disclosed in SEQ ID NO: 1 and a pharmaceutically acceptable carrier.

The therapeutically effective amount refers to an amount of an active material or formulation which produces a desired effect with a reasonable profit/risk ratio. The therapeutically effective amount refers to an amount necessary or sufficient to treat dry eye disease or symptoms of dry eye disease. The effective amount may vary depending on factors such as diseases or conditions to be treated, a specific composition to be administered, or severity of diseases or conditions.

The pharmaceutically acceptable carrier may be commonly used in formulation and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil and the like, but is not limited thereto.

The pharmaceutical composition of the present disclosure may further include a lubricant, a wetting agent, a sweeteners, a flavoring agent, an emulsifier, a suspending agent, a preservative, an isotonic agent, a buffer, a preservative, a surfactant, a lubricant, an excipient, a pH adjusting agent, and the like, in addition to the above components, but is not limited thereto.

Appropriate pharmaceutically acceptable carrier and preparation are described in detail in Remington: The Science and Practice of Pharmacy, (19th ed., 1995, Williams & Wilkins).

The pharmaceutical composition of the present disclosure may include a solution, a suspension, or a gel which is appropriate for topical administration to the eyes, including the peptide of the present disclosure and any appropriate carrier such as a saline solution or artificial tears.

The pharmaceutical composition of the present disclosure may further include one or more components known in the art of preparing a composition for topical administration to the eye.

The pharmaceutical composition of the present disclosure may be administered in all appropriate routes for treating or preventing dry eye disease, and for example, may be administered orally or parenterally, and in the case of parenteral administration, may be administered intravenously, subcutaneously, intramuscularly, intraperitoneally, topically, transdermally, and the like. The route of topical administration to the eye is preferred in that the pharmaceutical composition of the present disclosure is a composition for treating dry eye disease.

An administration amount of the pharmaceutical composition may be 0.0001 µg to 100 mg, 0.001 µg to 100 mg, 0.01 µg to 100 mg, 0.1 µg to 100 mg, or 1.0 µg to 1000 mg per day, but is not limited thereto, and may be variously prescribed depending on factors such as a formulation method, an administration method, patient's age, weight, sex, pathological condition, food, administration time, administration route, excretion rate, and reaction sensitivity.

According to a method which may be easily carried out by a person skilled in the art to which the disclosure pertains, the pharmaceutical composition of the present disclosure may be prepared into a unit dosage form by formulation using a pharmaceutically acceptable carrier and/or excipient or prepared by injection into a multi-volume vessel. Herein, the formulation may be in the form of a solution, a suspension, or an emulsion in an oil or aqueous medium, or an extract, powder, granule, tablet, or capsule form, and may further include a dispersing agent or a stabilizer.

In an exemplary embodiment, the pharmaceutical composition of the present disclosure has the following activities related to the tear secretion promoting activity:
(i) having a binding capacity to a pituitary adenylate cyclase 1 receptor (PAC1R) and promoting activation of PAC1R signaling pathway,
(ii) promoting movement of Aquaporin 5 (AQP5) protein to a plasma membrane, and
(iii) inducing neuroblast differentiation.

In an exemplary embodiment, the pharmaceutical composition of the present disclosure has an activity of expressing and secreting mucin which is a component of tears in conjunctival goblet cells.

In an exemplary embodiment, the pharmaceutical composition of the present disclosure has an activity of inhibiting inflammatory response and cell damage which are induced by IFN-γ in human corneal epithelial cells.

In an exemplary embodiment, the pharmaceutical composition of the present disclosure has an activity of inhibiting inflammatory response and cell damage which are induced by ultraviolet (UVB) irradiation in human corneal epithelial cells.

### Use of peptide

According to another aspect of the present disclosure, a use of a peptide comprising an amino acid sequence disclosed in SEQ ID NO: 1 for treating, preventing, or improving dry eye disease is provided.

According to another aspect of the present disclosure, a use of a peptide comprising an amino acid sequence disclosed in SEQ ID NO: 1 for preparing a medicament for treating, preventing, or improving dry eye disease is provided.

According to another aspect of the present disclosure, a method of treating, preventing, or improving dry eye disease comprising administering a peptide comprising an amino acid sequence disclosed in SEQ ID NO: 1 or a pharmaceutical composition comprising the peptide as an effective component to a patient or subject in need of treatment, prevention, or improvement of dry eye disease is provided.

The term "patient" or "subject" used in the present specification refers to human or non-human animals, such as human, primates, mammals, and vertebrates.

The descriptions for the peptide of the present disclosure, its activity, and the pharmaceutical composition thereof may be all identically applied to the use and the treatment method described above, and are not repeated in order to avoid excessive complexity of the specification.

### ADVANTAGEOUS EFFECT OF THE INVENTION

The peptide of the present disclosure has a binding capacity to PAC1R, promotes activation of a PAC1R signaling pathway, and induces neuroblast differentiation, thereby having a tear secretion promoting activity.

In addition, the peptide of the present disclosure promotes expression and secretion of mucin which is a tear component in conjunctival goblet cells, and has an activity of inhibiting inflammatory response and cell damage in human corneal epithelial cells.

The peptide of the present disclosure may be used as an active material for treatment, prevention, and improvement of dry eye disease.

However, the effect of the present invention is not limited to the effects mentioned above, and other effects which are not mentioned herein may be clearly understood by a person skilled in the art from the following description.

### BRIEF DESCRIPTION OF THE DRAWINGS

FIGS. 1A and 1B are results of SPR analysis showing that the peptide of the present disclosure has a binding capacity to PAC1R.
FIG. 2A is results of western blotting showing that the peptide of the present disclosure activates a PAC1R signaling pathway in human neuroblastoma cell line SH-SY5H to increase phosphorylation of PKA and CREB, and FIG. 2B is a graph showing a band intensity of the western blotting results of FIG. 2A as a relative ratio to a control group.
FIG. 3 is results of western blotting showing that the peptide of the present disclosure promotes the movement of AQP5 protein to a cell membrane in human neuroblastoma cell line SH-SY5Y.
FIG. 4 is results of western blotting showing that the peptide of the present disclosure increases an expression level of a mature neural marker GAP43 in human neuroblastoma cell line SH-SY5Y in a concentration-dependent manner.
FIG. 5 is experiment results showing that the peptide of the present disclosure increases MUC2 and MUC5AC gene expression levels in conjunctival goblet cells.
FIGS. 6A and 6B are results of western blotting and ELISA showing that the peptide of the present disclosure increases the expression level of MUC2 and MUC5AC proteins in conjunctival goblet cells.
FIG. 7A is experiment results showing that the peptide of the present disclosure inhibits an expression level of COX-2, IL-1β, and IL-6 genes which were increased by IFN-γ in human corneal epithelial cells, and FIG. 7B is a graph showing a band strength of FIG. 7A as a relative ratio to a control group.
FIG. 7C is experiment results showing that the peptide of the present disclosure inhibits an expression level of MMP3 and MMP9 genes which were increased by IFN-γ in human corneal epithelial cells, and FIG. 7D is a graph showing a band strength of FIG. 7C as a relative ratio to a control group.
FIG. 8A is experiment results showing that the peptide of the present disclosure decreases an expression level of COX-2, IL-1β, and IL-6 genes which were increased by UVB irradiation in human corneal epithelial cells, and FIG. 8B is a graph showing a band strength of FIG. 8A as a relative ratio to a control group.
FIG. 8C is experiment results showing that the peptide of the present disclosure inhibits an expression level of MMP3 and MMP9 genes which were increased by UVB irradiation in human corneal epithelial cells, and FIG. 8D is a graph showing a band strength of FIG. 8C as a relative ratio to a control group.
FIG. 9A is a schematic diagram of 5 areas of the cornea in which corneal damage was scored according to National Institute of Eye (NIE) guidelines when evaluating clinical corneal damage.
FIG. 9B is experiment results showing a tear film maintenance effect of the peptide of the present disclosure in a dry eye disease model mouse, and FIG. 9C is a table showing information on experimental group configuration, administered materials, and administered doses of the experiment results of FIG. 9B.
FIGS. 10A to 10C are experiment results showing a corneal damage improvement effect of the peptide of the present disclosure in a dry eye disease model mouse, and a table showing information on experimental group configuration, administered materials, and administered doses of the experiment, respectively.

### DETAILED DESCRIPTION

Hereinafter, the present disclosure will be described in detail by the examples. However, the following examples only specifically illustrate the present disclosure, and the content of the present disclosure is not limited by the following examples.

### Preparation Example 1: Preparation of peptide

Peptides having the amino acid sequence of SEQ ID NO: 1 described in the following Table 1 was synthesized using an automatic peptide synthesizer (Milligen 9050, Millipore, USA), and the synthesized peptides were separated into a pure form using C18 high performance liquid chromatography (HPLC, Waters Associates, USA). ACQUITY UPLC BEH300 C18 (2.1 mm×100 mm, 1.7 µm, Waters Co, USA) was used as the column.

**[Table 1]**

| SEQ ID NO: | Amino acid sequence (N-terminal → C-terminal) |
|---|---|
| 1 | TLKKNKRFSK |

The efficacy of the peptide of SEQ ID NO: 1 prepared above was evaluated by the following experiment.

### Experimental Example 1: Surface Plasmon Resonance (SPR) analysis

It was evaluated by SPR analysis whether the peptide of SEQ ID NO: 1 prepared in Preparation Example 1 has a binding capacity to a pituitary adenylate cyclase 1 receptor (PAC1R).

The analysis was performed using Biacore T200 (Cytiva, USA) equipment. Recombinant human PAC1R protein (LSBio, USA) was immobilized on CM5 chip (Cytiva, USA) using an amine coupling method. Samples in which the peptide was diluted by concentration with a 1X HBS-EP buffer which was a running buffer were prepared, and then the prepared samples were flowed onto the surface of the chip. An increase in response unit (RU) by concentration shown by binding of the peptide with concentration to PAC1R was analyzed using Biacore T200 evaluation software ver. 3.1 (Cytiva, USA).

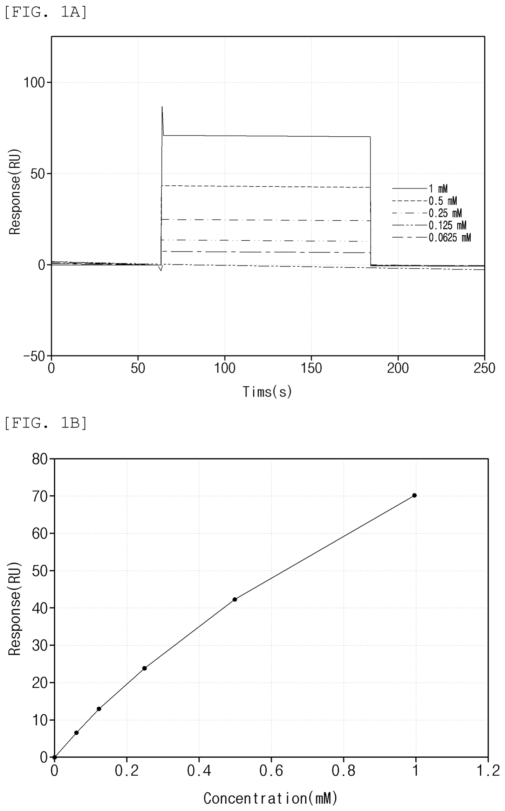

As a result of SPR analysis, it was confirmed that the peptide of SEQ ID NO: 1 showed an increase in RU value in a concentration-dependent manner, and the peptide has a binding capacity to PAC1R (FIGS. 1A and 1B).

### Experimental Example 2: Analysis of PAC1R signaling pathway activation

It was evaluated by a western blotting method whether the peptide of SEQ ID NO: 1 prepared in Preparation Example 1 activates the PAC1R signaling pathway.

Human neuroblastoma cell line SH-SY5Y was cultured for 24 hours by seeding in a 6-well plate at a density of 1.5×10⁶ cells/well. The cells were pretreated with PA8 which is a PAC1R antagonist at a concentration of 10 nM for 1 hour. Subsequently, the cells were treated with the peptide by concentration (10 µM, 50 µM, 100 µM) for 15 minutes, washed twice with PBS, and lysed by adding a cell lysis buffer. Treatment with a 5X sample buffer was performed, and then SDS-PAGE was performed using a 10% SDS-PAGE gel. Protein separated by SDS-PAGE was transferred to a PVDF membrane. Blocking was performed at room temperature for 1 hour using 5% skim milk. Subsequently, the primary antibody for p-PKA or p-CREB described in the following Table 2 was diluted in the 5% skim milk at a ratio of 1:1000 and reacted with the membrane for 2 hours. Washing with 0.1% PBS-T (0.1% Tween-20 in PBS) was performed 3 times for 10 minutes each. Subsequently, each corresponding secondary antibody described in the following Table 2 was diluted in 5% skim milk at a ratio of 1:3000 and reacted with the membrane for 1 hour. Treatment with an ECL solution (GE Healthcare, Cat. No.: RPN2232, USA) was performed, and then the expression level of p-PKA and p-CREB was analyzed using ImageQuant 800 (Cytiva, Cat. No: 29-3994-81, USA).

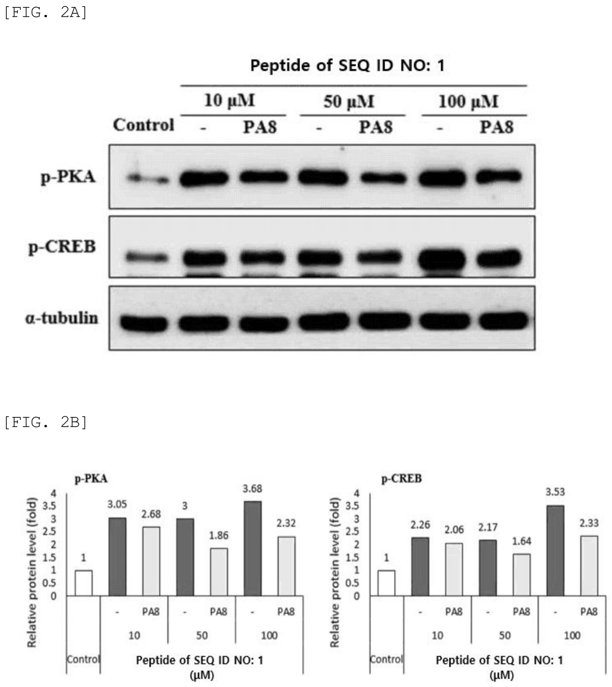

As a result of western blotting analysis as described above, it was confirmed that since the PAC1R signaling pathway was activated when treated with the peptide of SEQ ID NO: 1, phosphorylation of protein kinase A (PKA) and cAMP response element binding protein (CREB) was increased, and the increase in phosphorylation of PKA and CREB as such was decreased by the pretreatment with PA8 which is a PAC1R specific antagonist (FIGS. 2A and 2B). It was confirmed from the experiment results that the peptide of SEQ ID NO: 1 acted as an agonist for PAC1R.

### Experimental Example 3: Analysis of plasma membrane movement of Aquaporin 5 (AQP5) protein

It was evaluated by the western blotting method whether the peptide of SEQ ID NO: 1 prepared in Preparation Example 1 promotes movement of Aquaporin 5 (AQP5) protein to a plasma membrane, following the PAC1R signaling pathway activation.

Human neuroblastoma cell line SH-SY5Y was cultured for 24 hours by seeding in a 6-well plate at a density of 1.5×10⁶ cells/well. The cells were treated with the peptide by concentration (10 µM, 50 µM, 100 µM) for 1 hour and washed with PBS twice, and a protein sample was prepared using a plasma membrane protein extraction kit (Abcam, Cat. No.: ab65400, USA). Treatment with a 5X sample buffer was treated, and then SDS-PAGE was performed using a 10% SDS-PAGE gel. Protein separated by SDS-PAGE was transferred to a PVDF membrane. Blocking was performed at room temperature for 1 hour using 5% skim milk. The primary antibody for AQP5 of the following Table 2 was diluted in 5% skim milk at a ratio of 1:1000 and reacted with the membrane for 2 hours. Washing with 0.1% PBS-T (0.1% Tween-20 in PBS) was performed 3 times for 10 minutes each, and the secondary antibody of the following Table 2 was diluted in 5% skim milk at a ratio of 1:3000 and reacted with the membrane for 1 hour. Treatment with an ECL solution (GE Healthcare, Cat. No.: RPN2232, USA) was performed, and then the expression level of AQP5 protein was analyzed using ImageQuant 800 (Cytiva, Cat. No: 29-3994-81, USA).

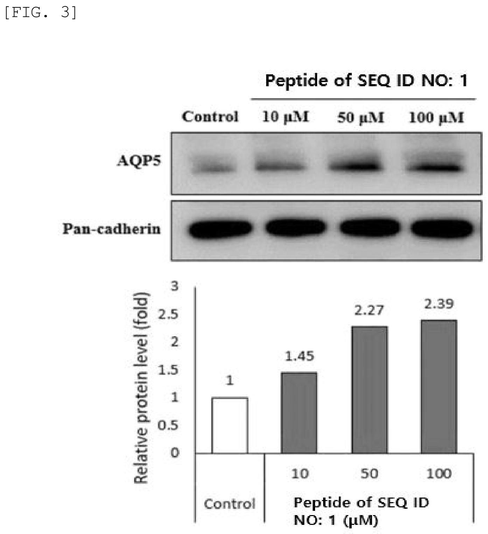

It is known that when the PAC1R signaling pathway is activated, tear secretion is occurred with the movement of AQP5 to the plasma membrane. According to a result of the above experiment, a concentration-dependent increase in the AQP5 protein was confirmed by the result of measuring the level of AQP5 protein found in the plasma membrane after treated with the peptide of SEQ ID NO: 1(see FIG. 3).

### Experimental Example 4: Analysis of neuronal differentiation

It was evaluated whether the peptide of SEQ ID NO: 1 prepared in Preparation Example 1 induced differentiation of neuroblast by measuring an expression amount of Growth Associated Protein 43 (GAP43) which is a mature neuronal marker.

Human neuroblastoma cell line SH-SY5Y was cultured for 24 hours by seeding in a 6-well plate at a density of 5×10⁶ cells/well. The cells were treated with the peptide by concentration (10 µM, 50 µM, 100 µM) for 24 hours, washed with PBS twice, and lysed by adding a cell lysis buffer. Treatment with a 5X sample buffer was treated, and then SDS-PAGE was performed using a 10% SDS-PAGE gel. Protein separated by SDS-PAGE was transferred to a PVDF membrane. Blocking was performed at room temperature for 1 hour using 5% skim milk. Subsequently, the primary antibody for GAP43 of the following Table 2 was diluted in the 5% skim milk at a ratio of 1:1000 and reacted with the membrane for 2 hours. Washing with 0.1% PBS-T (0.1% Tween-20 in PBS) was performed 3 times for 10 minutes each. Subsequently, the secondary antibody of the following Table 2 was diluted in 5% skim milk at a ratio of 1:3000 and reacted with the membrane for 1 hour. Treatment with an ECL solution (GE Healthcare, Cat. No.: RPN2232, USA) was performed, and then the expression level of GAP43 protein was analyzed using ImageQuant 800 (Cytiva, Cat. No: 29-3994-81, USA).

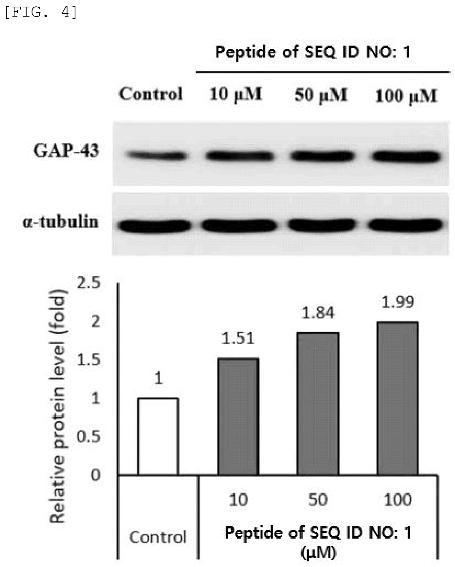

A pituitary adenylate cyclase-activating polypeptide (PACAP) which is a neuropeptide is a peptide hormone in the body, and VPAC1, VPAC2, and PAC1 receptors are known as the receptor of said peptide hormones. It is known that the neuroblast is differentiated by PACAP to increase synapse formation, thereby increasing secretion of tear film component factors (Int J Mol Sci. 2022 Jan 8;23(2):664.). As a result of the experiment, it was confirmed that when neuroblast was treated with the peptide of SEQ ID NO: 1, an expression level of GAP43 which is a mature neuronal marker was increased in a concentration dependent manner (see FIG. 4).

### Experimental Example 5: Analysis of expression level of mucin genes

It was evaluated whether the peptide of SEQ ID NO: 1 prepared in Preparation Example 1 increased the expression level of mucin genes in conjunctival goblet cells.

LS174T which is human colon adenocarcinoma cell line (a well-differentiated human colonic goblet cell line) was seeded in a 6-well plate at a density of 2×10⁶ cells/well and cultured for 24 hours. After culturing in a serum-free medium for 24 hours, the cells were treated with the peptide by concentration (10 µM, 50 µM, 100 µM) for 4 hours. The cells were washed twice with PBS, and RNA was separated using easy blue (iNtRON, Cat. No.: 17061, Korea). The separated RNA was quantified, and then cDNA was synthesized using a RT kit (enzynomics, Cat. No.: RT200, Korea). Subsequently, PCR was performed using a PCR kit (enzynomics, Cat. No.: P581T, Korea). The sequences of primers used in PCR are shown in the following Table 3.

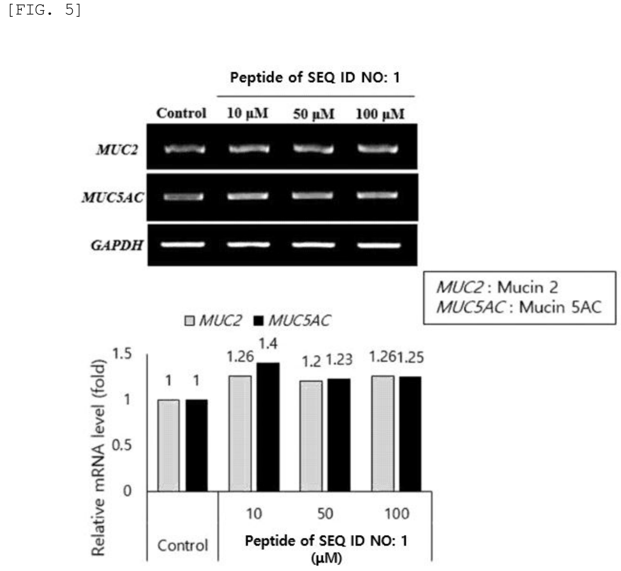

Mucin secreted in conjunctival goblet cells are another component forming a tear film. As a result of the experiment, it was confirmed that when the conjunctival goblet cells were treated with the peptide of SEQ ID NO: 1, expression levels of mucin genes of MUC2 (Mucin 2) and MUC5AC (Mucin 5AC) were increased (see FIG. 5).

### Experimental Example 6: Analysis of expression level of mucin protein

It was evaluated whether the peptide of SEQ ID NO: 1 prepared in Preparation Example 1 increased the expression level of mucin protein in conjunctival goblet cells.

LS174T which is human colon adenocarcinoma cell line (a well-differentiated human colonic goblet cell line) was seeded in a 6-well plate at a density of 1.5×10⁵ cells/well and cultured for 24 hours. After culturing in a serum-free medium for 24 hours, the cells were treated with the peptide by concentration (10 µM, 50 µM, 100 µM) for 24 hours. Cells were washed twice with PBS, and lysed by adding a cell lysis buffer.

First, western blotting of MUC2 protein was performed as follows. Cell lysate was treated with a 5X sample buffer was treated, and then SDS-PAGE was performed using a 10% SDS-PAGE gel. Protein separated by SDS-PAGE was transferred to a PVDF membrane. Blocking was performed at room temperature for 1 hour using 5% skim milk. Subsequently, the primary antibody for MUC2 of the following Table 2 was diluted in the 5% skim milk at a ratio of 1:1000 and reacted with the membrane for 2 hours. Washing with 0.1% PBS-T (0.1% Tween-20 in PBS) was performed 3 times for 10 minutes each. Subsequently, the secondary antibody of the following Table 2 was diluted in 5% skim milk at a ratio of 1:3000 and reacted with the membrane for 1 hour. Treatment with an ECL solution (GE Healthcare, Cat. No.: RPN2232, USA) was performed, and then the expression level of MUC2 protein was analyzed using ImageQuant 800 (Cytiva, Cat. No: 29-3994-81, USA).

In addition, the expression level of MUC5AC protein in the cell lysate was measured using a MUC5AC ELISA kit (LSBio, Cat. No.: LS-F38060, USA).

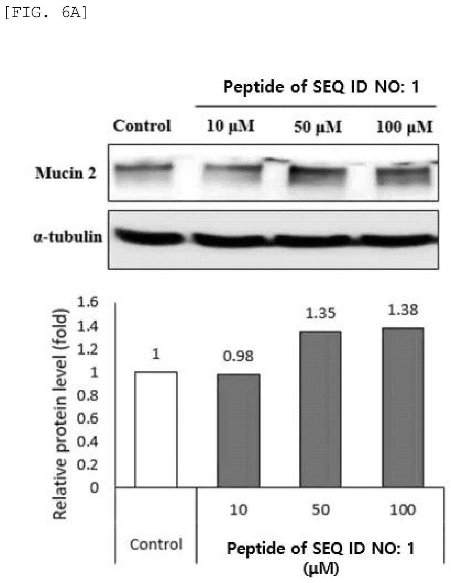

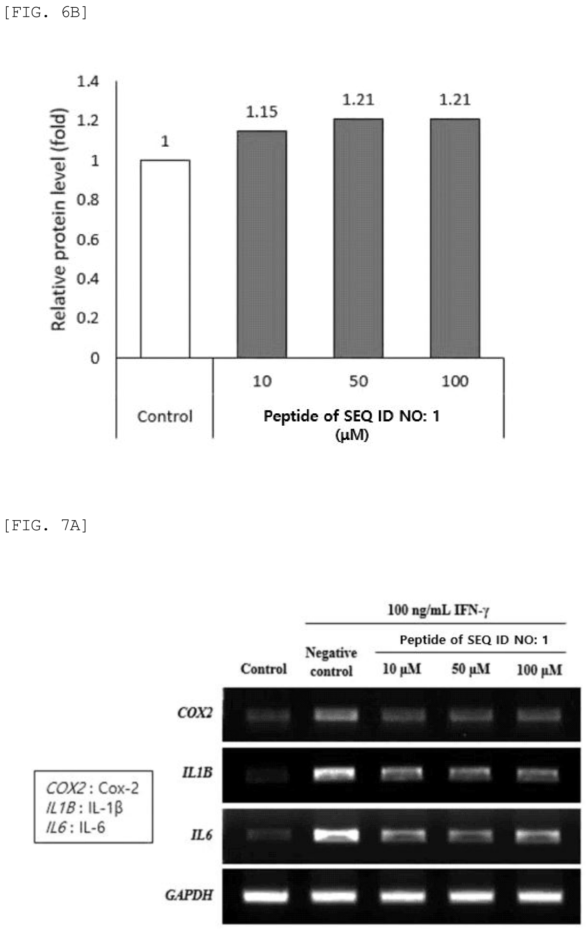

As a result of the experiment, it was confirmed that when goblet cells were treated with the peptide of SEQ ID NO: 1, the expression levels of MUC2 (Mucin 2) protein and MUC5AC (Mucin 5AC) protein were increased (FIGS. 6A and 6B).

### Experimental Example 7: Inhibition of inflammation and cell damage induced by IFN-γ in human corneal epithelial cells

It was evaluated whether the peptide of SEQ ID NO: 1 prepared in Preparation Example 1 has an activity of inhibiting cell damage induced by IFN-γ in human corneal epithelial cells.

Human corneal epithelial cells were cultured for 24 hours by seeding them in a 6-well plate at a density of 5×10⁵ cells/well. The peptide of SEQ ID NO: 1 was treated with the peptide by concentration (10 µM, 50 µM, 100 µM) for 24 hours. At this time, in the case of an induction group, it was also treated with 100 ng/mL of IFN-γ simultaneously. Subsequently, the cells were washed twice with PBS, and RNA was separated using easy blue (iNtRON, Cat. No.: 17061, Korea). The separated RNA was quantified, and then cDNA was synthesized using a RT kit (enzynomics, Cat. No.: RT200, Korea). Subsequently, PCR was performed using a PCR kit (enzynomics, Cat. No.: P581T, Korea). The primers used in PCR are shown in Table 3.

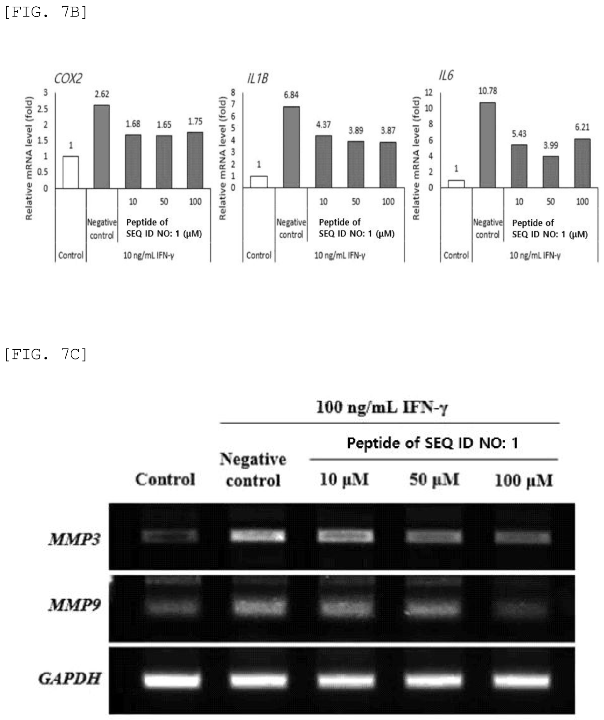

It is known that Th 1 cell activation after desiccating stress in the cornea and conjunctiva due to tear film destruction, and IFN-γ secretion promotion by Th 1 cell activation intensify damage to corneal and conjunctival tissues. According to the experiment results, it was confirmed that when the human corneal epithelial cells were treated with IFN-γ, an increase in the expression levels of COX-2, IL-1β, and IL-6 genes, which are pro-inflammatory molecules, was induced, and the increased expression level of genes of the pro-inflammatory molecules of COX-2, IL-1β, and IL-6 as such was inhibited by the peptide of SEQ ID NO: 1 (FIGS. 7A and 7B).

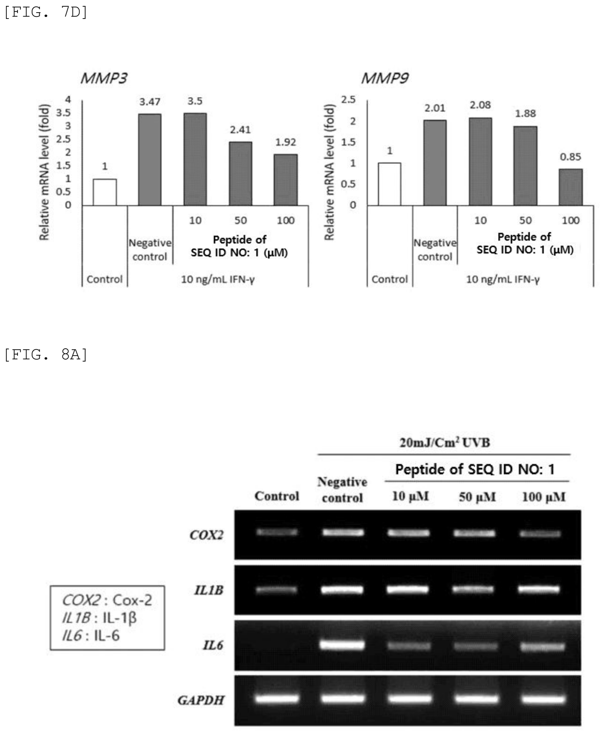

In addition, it was confirmed that when the human corneal epithelial cells were treated with IFN-γ, the expression levels of MMP (MMP3 and MMP9) genes were increased, and the increased expression level of MMP (MMP3 and MMP9) genes as such was inhibited by the peptide of SEQ ID NO: 1 (FIGS. 7C and 7D).

### Experimental Example 8: Inhibition of inflammation and cell damage induced by UVB in human corneal epithelial cells

It was evaluated whether the peptide of SEQ ID NO: 1 prepared in Preparation Example 1 has an activity of inhibiting cell damage induced by UVB in human corneal epithelial cells.

Human corneal epithelial cells were cultured for 24 hours by seeding them in a 6-well plate at a density of 5×10⁵ cells/well. After the medium was replaced with a serum-free medium, the peptide of SEQ ID NO: 1 was treated with the peptide by concentration (10 µM, 50 µM, 100 µM) for 1 hour. The cells were irradiated with 20 mJ/cm² UVB, and treated with the peptide of SEQ ID NO: 1 by concentration (10 µM, 50 µM, 100 µM) for 24 hours under serum-free medium conditions. The cells were washed twice with PBS, and RNA was separated using easy blue (iNtRON, Cat. No.: 17061, Korea). The separated RNA was quantified, and then cDNA was synthesized using a RT kit (enzynomics, Cat. No.: RT200, Korea). Subsequently, PCR was performed using a PCR kit (enzynomics, Cat. No.: P581T, Korea). The primers used in PCR are shown in Table 3.

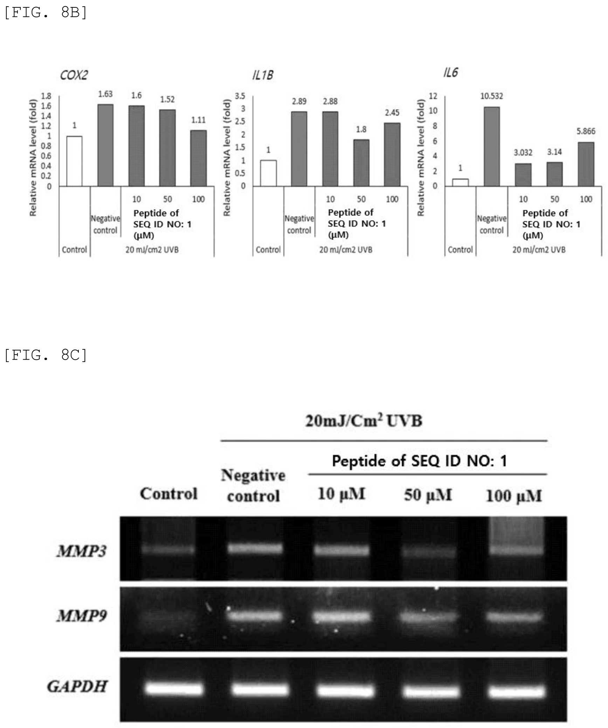

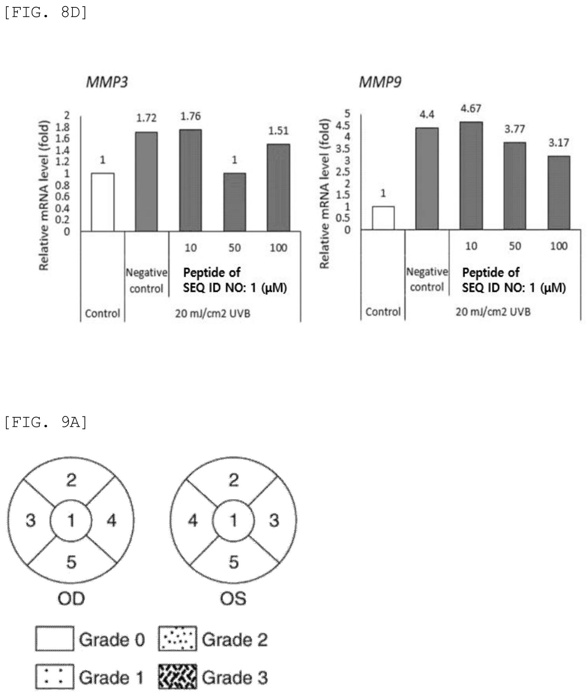

Ultraviolet (UV) irradiation is known as another stress factor which may induce dry eye disease. After inducing damage to corneal epithelial cells by ultraviolet (UV) irradiation, activation of improving cell damage of the peptide of SEQ ID NO: 1 was evaluated. As a result of the experiment, it was confirmed that the expression levels of COX-2, IL-1β, and IL-6 genes, which are pro-inflammatory molecules, increased after irradiating the human corneal epithelial cells with UVB and the increased expression levels of COX-2, IL-1β, and IL-6 genes were decreased again by the peptide of SEQ ID NO: 1 (FIGS. 8A and 8B). In addition, it was confirmed that when the human corneal epithelial cells were irradiated with UVB, the expression levels of MMP (MMP3 and MMP9) genes were increased, and the increased expression level of MMPs (MMP3 and MMP9) genes as such was decreased again by the peptide of SEQ ID NO: 1 (FIGS. 8C and 8D).

### Experimental Example 9: Activity of improving symptoms in dry eye disease mouse model

It was evaluated whether the peptide of SEQ ID NO: 1 prepared in Preparation Example 1 has an activity of improving dry eye disease symptoms in a dry eye disease mouse model.

### (1) Induction and maintenance of dry eye disease

While experiment animal (C57BL/6 mouse, 10 weeks old, female) was raised in a desiccating stress chamber at a relative humidity of 30% or less, scopolamine hydrobromide (Sigma-aldrich, Cat. No.: S0929, USA) prepared at a concentration of 2.5 mg/mL in saline water (0.9% normal saline, JW Pharmaceutical) was subcutaneously injected at a dose of 10 mL/kg 3 times a day for 10 days (TID, 9:30 am, 13:30 pm, and 17:30 pm) to cause dry eye disease. After starting administration of the test material, scopolamine was subcutaneously injected around the neck once daily (QD, 9:30 am) while the mouse was raised under the conditions of the relative humidity of 30% or less, in order to maintain the dry eye disease.

### (2) Preparation of administration material

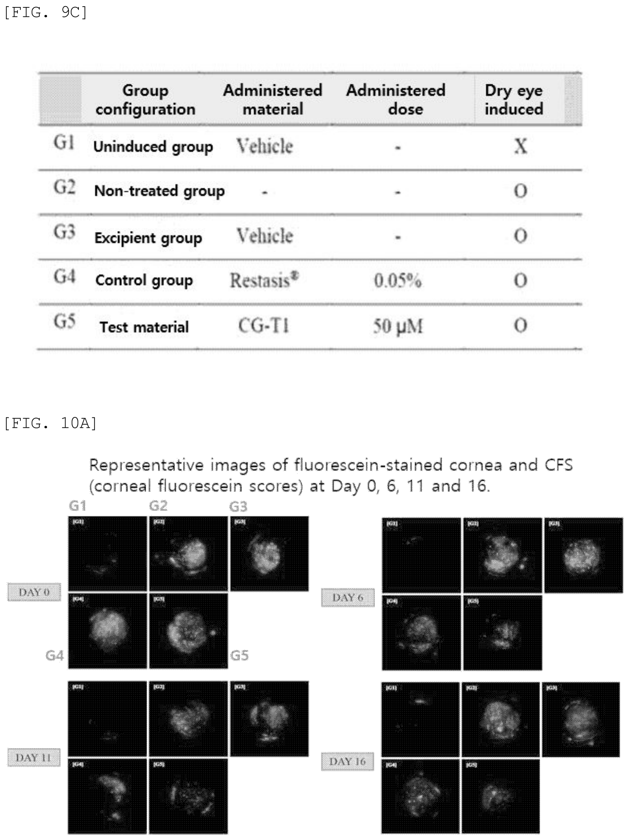

An experimental group was used in administration after the peptide of SEQ ID NO: 1 was dissolved in saline water (0.9% normal saline, JW Pharmaceutical) at a concentration of 50 µM, and filtered with a 0.22 µm filter (corresponding to CG-T1 in the table of FIG. 9C). As an excipient control group, the same saline water was filtered with a 0.22 µm filter and used in administration. As a positive control group, a 0.05% Restasis eye lotion (Restasis^{®}, Allergan) was administered.

### (3) Administration

One drop (5 µL) of each of the test material, the positive control material, and the excipient was administered by eye drop to both eyes with a micropipette twice a day (BID, 9:30 am and 17:30 pm) for 16 days. The number of subjects in each administration group was 7, and the number of eyes included in the final analysis was 12 to 14.

### (4) Evaluation of tear break-up time (TBUT) and evaluation of clinical corneal damage (corneal fluorescein score)

After inducing dry eye disease by desiccating stress and administration of scopolamine hydrobromide, general anesthesia in the mouse was induced at a time of group separation (Day 0), and 6 days, 11 days, and 16 days after administration of the test material, by intraperitoneally injecting 10 mg/kg of xylazine (Rompun^{®}, BAYER) and 75 mg/kg of Ketamine (Ketamine^{®}, Yuhan Corporation), and evaluation was performed.

Evaluation of tear break-up time (TBUT) was performed by the following method. First, a 0.4 M Fluorescein^{®} (Haag-streit AG, switzerland) test strip was wet with one drop of saline water and instilled in the lower conjunctival sac of the mouse, and the eyes were blinked 2 or 3 times to allow the fluorescence dye to sufficiently stain the tear film. The dyed eye was softly opened and a time when a first crack point or crack line form first appeared in the dyed tear film layer under blue light was measured in seconds.

Clinical corneal damage evaluation was performed by the following method. First, mydriasis was induced by dropping a mydriatic (TROPERIN^{®}, Alcon Korea) to the eye, and then 10 µL of each of 0.2% fluorescein sodium salt (FLUORESCITE^{®}, Alcon Korea) was dropped to both eyes to fluorescently stain the damaged area of the cornea. Subsequently, the eye was softly washed with saline water, the image of the cornea was taken (Micron-IV^{®}, Phoenix) under blue light, the area of the cornea was divided into 5 areas according to National Institute of Eye (NIE) guidelines, scoring was performed with 0-3 points for one area, and the values were summed to calculate the fluorescein score (FIG. 9A).

### (5) Experimental results

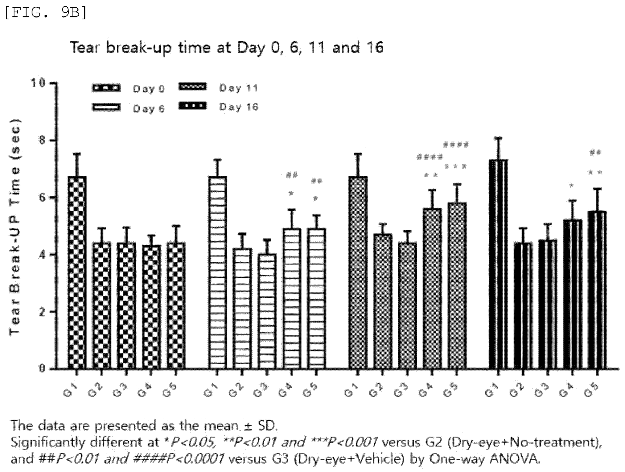

When the test material including the peptide of SEQ ID NO: 1 (CG-T1) at a concentration of 50 µM was administered to the dry eye disease model mouse twice a day(see FIG. 9C), a significant level of time extension was observed at day 6, day 11, and day 16 in the tear break-up time test as compared with a non-treated group and an excipient group. In particular, in the results at day 11 and day 16, higher significance was observed than the positive control group. From the experiment results, it was confirmed that the peptide of SEQ ID NO: 1 also showed the tear film maintenance effect even in a animal model of dry eye through controlling various mechanisms, the effect of which had been confirmed in the cell level experiment, resulting in the prevention of excessive tear evaporation (FIGS. 9B and 9C).

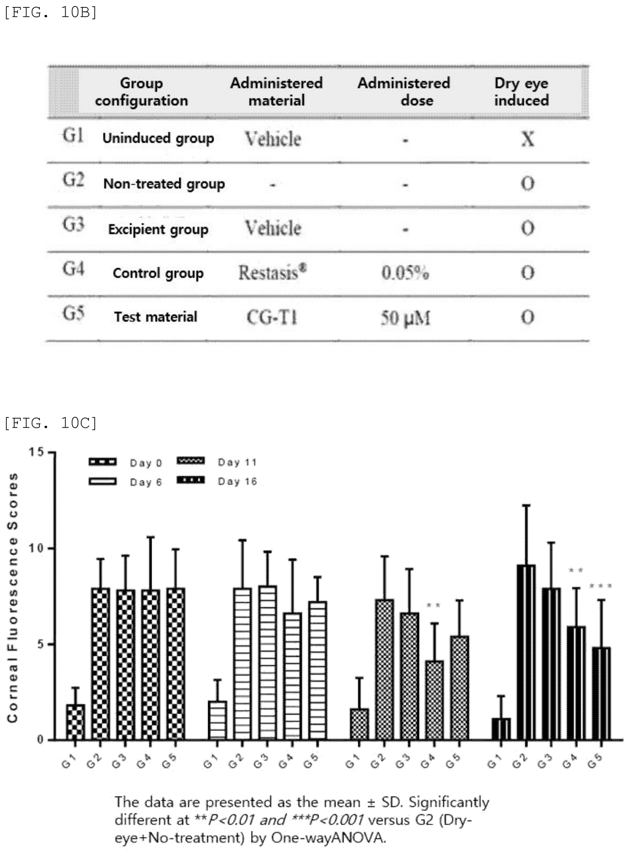

In addition, when the test material including the peptide of SEQ ID NO: 1 (CG-T1) at a concentration of 50 µM was administered to the dry eye disease mouse twice a day, the corneal fluorescence score increasing depending on corneal damage showed a decreasing trend at day 11 and decreased to a significant level at day 16, as compared with the non-treated group (see FIG. 10B). From the experiment results, it was found that the corneal damage by dry eye was improved to a significant level by the peptide of SEQ ID NO: 1 (FIGS. 10A to 10C).

**[Table 2]**

| Primary antibody | | | | Secondary antibody | | |
|---|---|---|---|---|---|---|
| Cell line | Antibodi es | Manufacturer | Cat. No. | Antibodies | Manufacturer | Cat. No. |
| | p-PKA | cell signaling technology (USA) | 4781 | Peroxidase-AffiniPure Goat Anti-Rabbit IgG (H+L) | Jackson ImmunoResearch (USA) | 111-035-003 |
| SH-SY5Y | p-CREB | cell signaling technology (USA) | 9198 | Peroxidase-AffiniPure Goat Anti-Rabbit IgG (H+L) | Jackson ImmunoResearch (USA) | 111-035-003 |
| | AQP5 | Santa Cruz Biotechnology (USA) | sc-514022 | Peroxidase-AffiniPure Goat Anti-Mouse IgG (H+L) | Jackson ImmunoResearch (USA) | 111-035-003 |
| | GAP-43 | Santa Cruz Biotechnology (USA) | sc17790 | Peroxidase-AffiniPure Goat Anti-Mouse IgG | Jackson ImmunoResearch (USA) | 115-035-003 |
| | | | | (H+L) | | |
| | α-tubulin | Santa Cruz Biotechnology (USA) | sc69969 | Peroxidase-AffiniPure Goat Anti-Mouse IgG (H+L) | Jackson ImmunoResearch (USA) | 115-035-003 |
| | Pan-cadherin | Santa Cruz Biotechnology (USA) | sc515872 | Peroxidase-AffiniPure Goat Anti-Mouse IgG (H+L) | Jackson ImmunoResearch (USA) | 111-035-003 |
| LS174T | Mucin2 | Santa Cruz Biotechnology (USA) | sc515032 | Peroxidase-AffiniPure Goat Anti-Mouse IgG (H+L) | Jackson ImmunoResearch (USA) | 115-035-003 |
| | α-tubulin | Santa Cruz Biotechnology (USA) | sc69969 | Peroxidase-AffiniPure Goat Anti-Mouse IgG (H+L) | Jackson ImmunoResearch (USA) | 115-035-003 |

**[Table 3]**

| Cell line | Primer | Sequence (5' → 3') | SEQ ID NO: |
|---|---|---|---|
| LS174T | MUC2 Forward | CGACTACTACAACCCTCCGC | 2 |
| | MUC2 Reverse | GGGAGGAGTTGGTACACACG | 3 |
| | MUC5AC Forward | AAGGTGGCTGACCAAGATGG | 4 |
| | MUC5AC Reverse | TCTCGTTTGTCATCACCCCG | 5 |
| | COX2 Forward | ATCATTCACCAGGCAAATTGC | 6 |
| | COX2 Reverse | GGCTTCAGCATAAAGCGTTTG | 7 |
| | IL-1β Forward | TTCGACACATGGGATAACGA | 8 |
| | IL-1 β Reverse | TCTTTCAACACGCAGGACAG | 9 |
| | IL-6 Forward | AAAGAGGCACTGCCAGAAAA | 10 |
| Human Corneal Epithelial Cell | IL-6 Reverse | ATCTGAGGTGCCCATGCTAC | 11 |
| | MMP3 Forward | CAAAACATATTTCTTTGTAGAGGACAA | 12 |
| | MMP3 Reverse | TTCAGCTATTTGCTTGGGAA | 13 |
| | MMP9 Forward | GGGACGCAGACATCGTCATC | 14 |
| | MMP9 Reverse | TCGTCATCGTCGAAATGGGC | 15 |
| | GAPDH Forward | GGAGCCAAAAGGGTCATCAT | 16 |
| | GAPDH Reverse | GTGATGGCATGGACTGTGGT | 17 |

Hereinabove, representative examples of the present disclosure had been illustratively described, but the scope of the present disclosure is not limited to the certain examples described above, and appropriate modification may be carried out within the scope of the claims of the present disclosure by a person with ordinary skill in the art.

## Claims

1. A peptide comprising an amino acid sequence of SEQ ID NO: 1.

2. A pharmaceutical composition for treating or preventing dry eye disease comprising the peptide of claim 1 as an effective component.

3. The pharmaceutical composition of claim 2, wherein the pharmaceutical composition has an activity of promoting tear secretion by one or more activities of the following activities:
(i) having a binding capacity to a pituitary adenylate cyclase 1 receptor (PAC1R) and promoting activation of PAC1R signaling pathway;
(ii) an activity of promoting movement of Aquaporin 5 (AQP5) protein to a plasma membrane; and
(iii) an activity of inducing neuroblast differentiation.

4. The pharmaceutical composition of claim 2, wherein the pharmaceutical composition has an activity of promoting expression and secretion of mucin in conjunctival goblet cells.

5. The pharmaceutical composition of claim 2, wherein the pharmaceutical composition has an activity of inhibiting inflammatory response and cell damage in human corneal epithelial cells.

6. The pharmaceutical composition of claim 2, wherein the dry eye disease includes one or more diseases, disorders, or symptoms selected from the group consisting of evaporative dry eye, water-deficient dry eye, dry keratitis, keratoconjunctivitis sicca, and dysfunctional tear syndrome.

7. The pharmaceutical composition of claim 2, wherein the pharmaceutical composition includes a solution, a suspension, or a gel appropriate for topical administration to the eye.
